# EUROPEAN PATENT APPLICATION

(11) **EP 3 991 771 A1**
(43) Date of publication of application: **04.05.2022**
(21) Application number: 20204580.3
(22) Date of filing: 29.10.2020
(51) Int. Cl.: A61M 5/142, A61M 5/20, G16H 20/17, G16H 80/00, A61M 5/32

(54) **DRUG DELIVERY DEVICE WITH INCREASED SAFETY**

(71) Applicant: Ypsomed AG, 3401 Burgdorf (CH)
(72) Inventor: Schneider, Andreas, 3027 Bern (CH); Urbanek, Leos, 3012 Bern (CH); Jost, Reto, 3506 Grosshöchstetten (CH)
(74) Representative: Meier Obertüfer, Jürg

(57) **Abstract**

The invention is concerned with a disposable, single-dose delivery device for self-administration, by a patient, of a predefined amount or dose of drug from a reservoir through an outlet of the reservoir. The delivery device has a device operation lock to prevent a delivery operation of the delivery device as shipped to the patient., an embedded electronic control unit with a wireless receiver for receiving an unlock command or message, and an electromechanical actuator for mechanically unlocking the device operation lock and enabling delivery device operation by the patient. The control unit is configured to activate the actuator instantaneously and unconditionally upon receipt of the unlock command. The unlocking mechanism of the delivery device is adapted to be activated from remote in response to a confirmative message indicative of additional double check by a Health Care Professional HCP or expert system.

## Description

### FIELD OF THE INVENTION

The present invention relates to medicament delivery devices for delivering, administering, dispensing, injecting, or infusing substances and/or liquids such as insulin or hormone preparations. It departs from a delivery device for self-administration of a predefined amount of drug from a reservoir.

### BACKGROUND OF THE INVENTION

A variety of diseases exist that require regular treatment by parenteral subcutaneous or intramuscular administration of a drug or medicament, and a number of drug delivery devices have been developed to support a patient in accurately and controllably delivering an amount of drug in a self-administration process. Delivery devices include *injection* devices that are removed from the injection site after each medication event or drug delivery process. An *injection pen* device has an elongate device body defining a longitudinal main device axis. The term "distal end" refers to the end of the injection device where an injection needle or cannula is located, the term "proximal end" designates the opposite end thereof. Delivery devices further include *infusion* devices or pumping devices for continual delivery of a regular or basal amount of medication through a fluid channel into the body of the patient, wherein the fluid channel remains in use for a prolonged period of time.

*Disposable* or single-use delivery devices are adapted to deliver a drug from a container such as a pre-filled syringe that is not intended to be replaced or refilled by the patient. *Reusable,* semi-reusable, or hybrid delivery devices have a container that may be replaced by the patient, or a cartridge that may be refilled, while some components of the device may be reused with the replaced or refilled drug container. An *automatic* injection device has an electric motor or a drive spring for biasing a piston rod and shifting a piston in a container barrel, wherein the drive spring may have to be charged or strained manually prior to injection of a dose. A *manually* powered injection device requires a user to manually provide the energy to move the piston, for instance by applying a distal force component to the injection device.

Drug delivery device based therapies generally benefit from an electronic unit or control unit embedded or integrated in the delivery device. The electronic unit monitors a drug delivery process, in order to proactively prevent false handling of the device and/or to keep track of the doses already applied, and generates data related to an instantaneous condition and/or use of the delivery device. Suitable sensors of the electronic unit readily detect a status or signal from any kind of indicating component of the delivery device, including user interface elements and actuators. A wireless communication unit of the electronic unit is provided to wirelessly communicate, specifically upload, drug delivery information to a nearby mobile device or dedicated medical gateway. The drug delivery information includes at least a time stamp and the expelled dose, indicative of a time of a medication event and of a quantity of delivered medicament. The drug delivery information may be transmitted instantaneously, or stored in a memory unit connected to the processing unit, for later upload or batch transfer.

EP 3013390 B1 discloses a bolus-only infusion pump that enables delivery of insulin via a mechanical drive mechanism that is controlled by the patient. In order to deliver a bolus the patient has to enter the amount into a remote controller. Subsequently, the controller sends a message to the pump that actuates a motor to unlock the mechanical drive mechanism. The patient dials the number of clicks at the pump to deliver medication. Once the desired amount is delivered a locking mechanism automatically engages disabling further delivery of medication.

EP 2879735 B1 discloses a cassette unit for an electrically powered auto-injector with a re-useable drive unit. The cassette unit is adapted to prevent accidental or unintended removal of a removable front-cap from the cassette unit by means of a shuttle lock control movable from a first 'cassette unused' position to a second 'cassette unlocked' position, in which it no longer prevents such cap removal. The shuttle lock control is configured to be manually releasable or automatically releasable by interaction with a suitable feature of the drive unit when the cassette unit locates at the docking position within the drive unit housing.

US 8,882,722 B2 discloses a reusable injection pen with a protective cap that is releasably retained over a distal end of a cartridge housing. A lockout feature is positioned between the cap and a dose setting mechanism and is configured to time lock the cap to the dose setting mechanism to prevent an authorized injection. The lockout feature is settable wirelessly from a remote location, to open or close at a specific time of day, or day of the week, or week of a month, according to a schedule pre-programmed by a Health Care Professional. After an injection has been made, the user can replace the replaceable cap to re-cover the cartridge housing. Once replaced a time lock will lock the cap to the dose setting mechanism until it is time for the next injection.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

### SUMMARY OF THE INVENTION

It is an objective of the invention to increase therapeutic safety and patient comfort in self-administration of drugs by means of a drug delivery device operated by a patient, while maintaining a certain level of flexibility. This objective is achieved by a device and a method according to the independent claims. Preferred embodiments are evident from the dependent patent claims.

According to the invention, a disposable, single-dose delivery device for self-administration, by a patient or user, of a predefined amount or dose of drug from a reservoir through an outlet of the reservoir, is adapted to prevent or inhibit a delivery operation of the delivery device as shipped to the patient. The delivery device has a device operation lock, an embedded electronic control unit with a wireless receiver for receiving an unlock command or message, and an electromechanical actuator for mechanically unlocking the device operation lock and enabling delivery device operation by the patient. The control unit is configured to activate the actuator instantaneously and unconditionally upon receipt of the unlock command. The unlocking mechanism of the delivery device is adapted to be activated from remote in response to a confirmative message indicative of a supplemental or double check by a Health Care Professional HCP or expert system. Receipt of the unlocking message is a necessary condition for a delivery operation, and introduces an additional layer of therapeutic safety and patient comfort. Unlocking of the device operation lock commences instantaneously and without delay, and does not depend on pre-defined schedules or criteria applied to actual environmental conditions at the place of the patient.

In a preferred embodiment, the delivery device is an auto-injector or a patch -injector, with a drive means such as a preloaded spring, for automated delivery, upon operation of a patient-operable trigger element, of a predefined, non-user selectable amount of drug. The amount of drug delivered is preferably the entire volume of the reservoir, but in exceptional circumstances, the device might be limited to deliver a predefined fraction, with the rest being discarded after use. The auto-injector has a needle cover sleeve as a trigger element and, preferably, no needle or syringe movement, while the patch-injector has a cannula insertion mechanism activated by a trigger button. The invention may also be beneficially applied to a multi-variable-dose injection device for repeated delivery of variable doses of drug from a container that may or may not be intended to be replaced by the patient. In this case the device locking mechanism is adapted for multiple operations, i.e. capable of re-locking at the end of a dose delivery operation.

In a preferred embodiment, the device operation lock includes an outlet cover lock preventing removal of a non-replaceable outlet cover sealing, and maintaining sterile and uncontaminated, the outlet of the reservoir. Specifically, the outlet lock is a device cap lock of the auto-injector preventing removal of a device cap and/or needle shield of the auto-injector, or a barrier lock of the patch injector preventing removal of a sterile barrier and/or release liner of the patch-injector. The device operation lock thus prevents the outlet from being exposed to ambient in the first place, such that the delivery device remains sterile and may be used at a later time. In a less preferred embodiment, the outlet cover is adapted to be removed and the device operation lock is adapted to lock the drive mechanism or trigger element instead, such that a locked device would have to be discarded in case the user inadvertently removes the outlet cover in the first place.

In a refined variant the electronic unit includes a timer and is configured to control the electromechanical actuator to mechanically re-lock or activate the device operation lock after lapse of a certain unlock delay or interval. Operation of the sterile and safe-to-use device is then disabled until a next unlock command is received. Re-locking may also take place in other cases, for instance if and/or as long as wireless communication to or from the delivery device is interrupted.

In a preferred embodiment, the device cap lock includes a flexible ledge engaging, in a locked or shipping state, a rigid counter-ledge, and prevented by a locking slider from disengaging the counter-ledge. One of the ledges is part of the device cap while the other is part of the device housing. The locking slider conveys an unlocking movement of the actuator to or towards the device operation lock, such that the actuator may be located at a distance from the latter, specifically in a proximal or rear part of the device. The unlocking movement of the locking slider may include an axial hub of 1 to 5 mm along a main axis of the delivery device.

In a preferred embodiment the electronic unit includes a wireless transmitter to send an unlock request message to the HCP or expert system, triggered by a user input or activity detected by the delivery device. The HCP or expert system may respond automatically with the unlock command if and as soon as the necessary conditions or requirements are deemed to be satisfied by the HCP or expert system. The unlock request message may also help to establish that a communication link to a therapy management system is in fact active and available for transmission of the expected unlock command.

In a refined variant the electronic unit includes a device-activation sensor operative at no or lowest power consumption during storage and shipping of the delivery device, and adapted to detect a user-initiated preparation of the delivery device indicative of a forthcoming operation of the device. The device-activation sensor means may include a touch sensor, a motion sensor, a temperature sensor, or an electromechanical switch coupled to and activated by a device packaging lid or cover. The electronic unit is configured to activate or wake-up from an idle or stand-by state based on a corresponding response of the device-activation sensor, and initiate the unlock request message transmission.

In a preferred embodiment the electronic unit of the delivery device includes a drug delivery sensor such as the rotation sensor disclosed in the patent application CH 715791 A2 to monitor a drug delivery operation of the device. The electronic unit is further adapted to produce a time-stamp indicating at what date and time a monitored dose has been dispensed, or to start a counter indicative of how long ago the dose dispense has occurred. The electronic unit is adapted to store delivery data including the dose delivered, the time-stamp, and a quality of the delivery including holding time in a data storage unit, and to upload the latter and/or a counter value at upload time to the HCP or other stakeholder. The electronic unit of the delivery device may further include a device status indicator that provides visual feedback about a device or process status including an availability of battery power, a readiness of communication means, or a progress of an ongoing delivery process.

In a preferred embodiment, the actuator includes a shape-memory-alloy element in the form of a wire, which is capable of producing the required hub without additional mechanical gearing. Preferably, the shape memory alloy wire has a diameter of 0.01-1 mm, specifically 0.05-0.1 mm, and/or a length of 30-100 mm, specifically 50-70 mm, and may develop a hub of 1-5 mm, specifically 1.5-3 mm within a few seconds when heated to a critical temperature. The wire dimensions in the range indicated allow a reasonably sized energy source or battery to provide the necessary heating power.

According to the invention a method of safely injecting, by way of self-administration, a predetermined quantity of drug from a reservoir through an outlet of a delivery device with a device operation lock preventing a delivery operation of the delivery device as shipped, comprises the steps of:
- establishing a communication or contact between the patient and a remote Health Care Professional HCP via telephone or video call, or between the patient and a Health Care Expert System HCES via email or short message text, and inquiring about an intended self-administration;
- confirming, or approving, by the HCP or HCES, in particular following an anamneses of the patient by the HCP and/or a check of therapy plan and device data by the HCES, safety and suitability of the intended self-administration, and authorizing the latter by dispatching an unlock command message;
- receiving, by a wireless receiver of the delivery device, the unlock command, and
- unlocking, by an actuator of the delivery device and instantaneously upon receipt of the unlock command, the device operation lock.

The confirmation by the HCP and/or the HCES is part of an integrated therapy management and allows to monitor or supervise the self-administration of a dose of drug by a remotely located qualified person or entity. In particular, the HCP may proceed to an anamnesis of the patient, to ensure that the patient is in a physical condition to receive the medication, without the patient and/or HCP having to travel. The HCES may proceed to an automated check of the specified delivery device, including verification of the intended drug and dose, expiry date, absence of device recall, and correct storage temperature. The patient is ultimately assured that he or she is about to proceed to the correct administration by being able to use the unlocked device, specifically by being able to remove an outlet cover.

In a preferred embodiment, the method comprises the steps of
- assigning, or linking, the delivery device as identified by a Unique Device Identifier UDI, or a package of plural identical delivery devices, to the patient, which may occur by the pharmacy or distributor supplying the identified device or devices to the patient, or by the patient himself or herself; and, at a therapy management system, recording or registering such assignment, and
- dispatching the unlock command, by the therapy management system, to or for a specific delivery device based a confirmation or approval from the HCP or HCES, and based on the recorded assignment of the delivery device or devices to the patient.

In a preferred embodiment, the method comprises the steps of
- detecting, by a device-activation sensor of the delivery device, a user-initiated preparation or handling of the delivery device indicative of an intended or forthcoming drug delivery operation of the device,
- sending, by a wireless transmitter of the delivery device, an unlock request message to the HCP or HCES, and
- contacting, by the HCP or HCES, the patient, and inquiring about the patient condition to confirm or approve safety and suitability of the intended self-administration, by the HCP.

### BRIEF DESCRIPTION OF THE DRAWINGS

The subject-matter of the invention will be explained in more detail in the following text with reference to preferred exemplary embodiments as illustrated in the attached drawings, of which
Fig.1 depicts a medical injection monitoring and patient support system;
Fig.2 is a flow chart of an exemplary method of safely unlocking a delivery device;
Figs.3a to 3c depict an auto-injector, a locking slider, and an unlocking actuator of a first embodiment; and
Figs.4a to 4c illustrate three variants of the locking slider - device cap interaction.

For consistency, the same reference numerals are used to denote similar elements illustrated throughout the drawings.

### DETAILED DESCRIPTION OF PREFERRED EMBODIMENTS

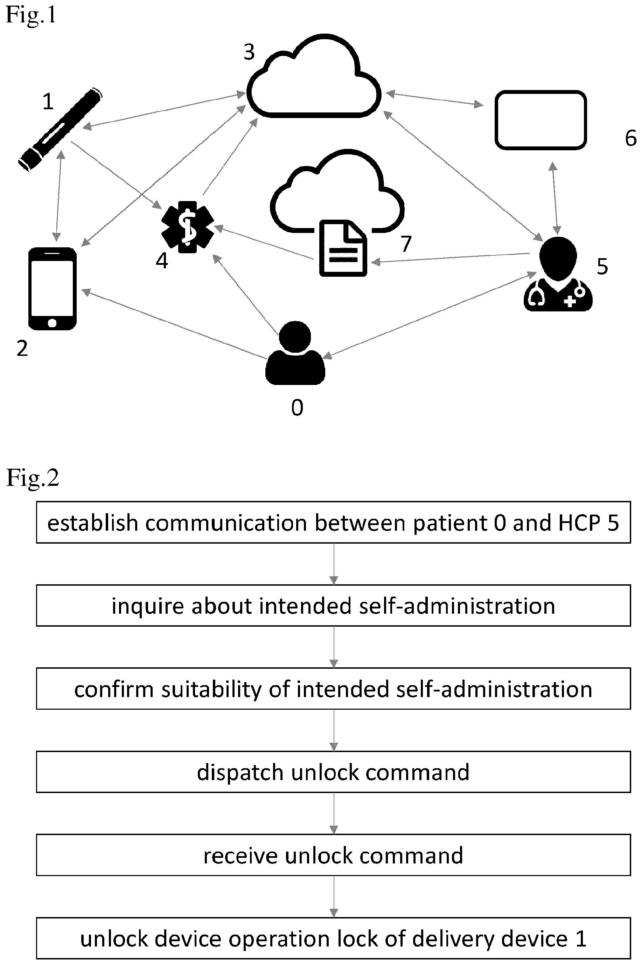

Fig. 1 depicts a medical drug delivery monitoring and patient support system including a delivery device 1 for self-administration of a dose of drug to a patient 0. A smartphone of the patient serves as a user and/or device gateway 2 to a therapy management system 3. A pharmacy 4 is adapted to sell and supply the delivery device 1 to the patient and to provide an association or link between delivery device 1 and patient 0 to the therapy management system 3. A Health Care Professional (HCP) 5 such as a physician is enabled to interact with the patient 0 and the therapy management system 3, optionally via a dedicated tele-health portal or interface 6. An electronic patient file 7 comprising therapy relevant information and/or specific recipes of the patient 0 may be available for reading and writing by the pharmacy 4 and the HCP 5.

The delivery device 1 is a disposable auto-injector with an elongate, pen-shaped device housing essentially symmetric around a main device axis, and a patient-operable trigger element for triggering automated delivery of the dose of drug. The delivery device may also be a disposable patch injector, or wearable bolus injector, with an essentially flat shape factor and with a skin adhesive layer for injection of the dose over prolonged periods of up to 15 minutes. The delivery device may also be a manual device with a delivery lever or button for drug delivery through a force provided by the user.

The delivery device integrates an electronic unit with a short-range communication facility such as Bluetooth Low Energy (BLE) or equivalent short or near range wireless communication technology, such as NFC, WiFi, or IR, to communicate with a nearby gateway device 2 such as a mobile device of the user, including a smartphone or tablet device running a dedicated application program, or a laptop computer configured accordingly. The gateway device 2 in turn communicates with a therapy management system 3 on a cloud-based, distributed data server or computing facility, by way of a 4G/LTE cellular mobile and/or wire-based communication network, and is adapted to relay data between the delivery device 1 or the patient and the therapy management system. Alternatively, the communication facility of the delivery device 1 may communicate directly with the therapy management system 3 via wireless long-range communication networks such as 5G cellular mobile networks, nb-IoT, LTE-m, LoRa, Sigfox, and the patient 0 may interface the data servers of the therapy management system 3 via other communication means. All communication is state-of-the-art encrypted, authenticated and/or otherwise secured in order to comply with relevant regulations applicable to protected health information.

The therapy management system 3 is configured to handle device unlocking requests from the delivery device 1 and unlocking approvals from the HCP 5. To that end, the system receives and registers, in a user account of the patient 0, an assignment or association between the patient 0 and the delivery device 1, either from the pharmacy 2 supplying the device, or from the patient, e.g. by recording and transmitting a Unique Device Identification UDI of the device in the form of printed text, an optical code, or an RFID tag. The UDI includes more generic information about the package in which the specific device was supplied, the production batch, and the drug type, quantity, concentration, and expiry date. The therapy management system may be adapted to store delivery data, drug information, patient adherence data, or any further monitoring data accumulated by adequate sensors of the electronic unit of the delivery device 1 and uploaded to the data server of the therapy management server 3.

The HCP and pharmacy have access to the electronic file of the patient, which comprises at least the prescription for the current therapy, and which may be stored in or next to the user account of the patient on the data server of the therapy management system. The HCP has access to the therapy management system and the registered assignments, or is otherwise given the authority, to have the system dispatch unlock commands based on unlocking approvals from the HCP. The unlock approval of the HCP may be indicating i) any delivery device with the correct drug type, quantity, and concentration as specified, ii) any one device of a production batch as specified, iii) any one device of a package or box of plural devices as specified, or iv) a specific delivery device. The therapy management system is configured to identify, in cases i) to iii), a specific delivery device available to the patient for the intended administration, and to send an unlock command for or to precisely this device.

Fig.2 is a flow chart summarizing essential steps of an exemplary method of safely unlocking a delivery device with a device operation lock.

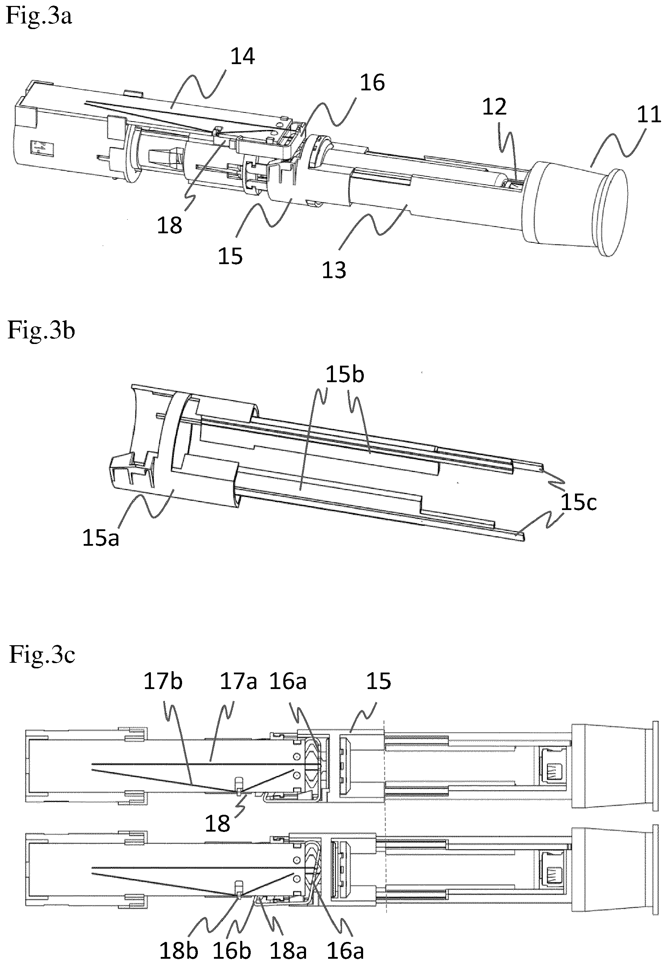

Fig.3a depicts a perspective view of an auto-injector device 1 according to a first embodiment, in an initial or storage state with a device cap 11 for removing a needle shield of a prefilled syringe 12 being mounted to the device. The housing of the auto-injector has been omitted in order to reveal the axially movable cover sleeve 13 as the trigger element and a drive unit at a proximal end of the device. The latter includes an electronic unit 14 with a Printed Circuit Board PCB mounted immovably in the device and oriented parallel to the main axis of the device. The PCB accommodates sensors for sensing a status of the device and communication means for transmitting and receiving device and/or operation data to or from a nearby mobile device of the patient or a remote expert. The electronic unit 14 also includes an actuator for a cap unlocking mechanism with a shape memory alloy wire arranged on the outer side of the PCB. The actuator is connected to a locking slider 15 that essentially extends from the electronic unit 14 to the device cap 11 and may be shifted axially back and forth a distance corresponding to an actuating hub. A proximal end of the locking slider 15 is axially fixed to a bracket 16 of the actuator and a distal end is adapted and/or arranged to interact with the cap unlocking mechanism. A major part of the locking slider 15 is radially arranged between the cover sleeve 13 and a syringe holder, a proximal flange of which acts as a guiding means for the locking slider 15.

Fig.3b depicts the locking slider 15 of Fig.10a in an isolated view. The locking slider comprises a part-cylindrical base 15a at a proximal end and two arms 15b extending therefrom in distal direction. The two arms 15b are radially closer to the center of the device axis and obscured by the cover sleeve 13 in the view of Fig.3a. A radial blocking surface at the tip or distal end 15c of the arms interacts with the cap unlocking mechanism as detailed below.

Fig.3c depicts a top view of the unlocking actuator in the locked state (top) and in the unlocked state (bottom). A first or unlocking shape memory alloy wire 17a is arranged parallel to the main axis, with a proximal end thereof fixed to a first electrical contact on the PCB, and with a distal end thereof fixed to the metallic actuator bracket 16 as a second electrical contact. Specifically, the distal end is connected to an axially flexible actuator arm 16a of the bracket, which is biased and in turn biases the locking slider 15 in the distal direction by virtue of the elastic properties of the metallic bracket 16. For actuating the cap unlocking mechanism, a current is passed through the first wire 17a, which causes the wire to heat up to a critical temperature at which phase change sets in, and to correspondingly reduce its length by 2-5%. By way of example, a commercial shape memory alloy wire with a diameter of 0.05-0.1 mm and a length of 50-70 mm may develop a hub of 1.5-3 mm within 1-2 seconds when heated to a temperature of 70° or higher by a current of 100-200 mA This contraction in turn pulls the actuator arm 16a and the locking slider 15 in a proximal direction against the bias mentioned. A transverse ledge 16b of the actuator arm 16a engages a proximally oriented blocking surface 18a or edge of a transversally movable anchor 18. Such engagement prevents the locking slider 15 in the unlocked state from shifting distally under the bias of the flexible bracket arm. The actuator includes a second, or locking, shape memory alloy wire 17b connected at both ends to corresponding contacts and forming a kink or deviation angle at an intermediate pulley 18b of the anchor 18. For actuating a cap locking mechanism, or for de-actuating the cap unlocking mechanism, a current is passed through the second wire 17b, which in turn contracts and pulls the intermediate pulley 18b and the anchor 18 in a transverse direction in an attempt to decrease the deviation angle. The transverse ledge 16b passes out of engagement with the blocking surface 18a of the anchor 18, and the actuator arm 16a and locking slider 15 move distally under the distal bias of the elastic bracket 16. Alternatively, the second wire may be arranged to urge the transverse ledge 16b out of engagement with a stationary blocking surface.

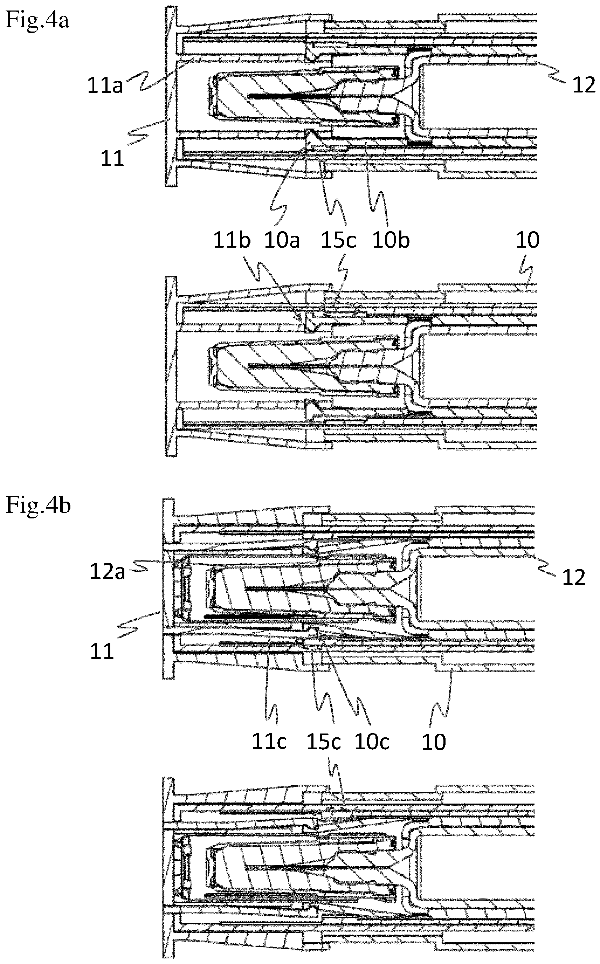

Fig.4a depicts a longitudinal section along the main axis of the auto-injector with a first variant of the locking slider - device cap interaction in the locked state (top) and in the unlocked state (bottom). An inner sleeve 11a of the device cap 11 comprises a recess 11b engaged in the locked state by a locking ledge 10a at a distal end of a flexible locking arm 10b integral with the housing 10. A distal tip 15c of the locking slider 15 prevents the locking ledge 10a from radial movement and from disengaging the recess 11b. In the unlocked state the locking slider 15 has moved proximally by an unlocking hub allowing the locking ledge 10a to flex radially, by virtue of an inclined contact surface at the locking ledge 10a and/or the recess 11b, when the device cap 11 is pulled distally.

Fig.4b depicts a longitudinal section along the main axis of the auto-injector with a second variant of the locking slider - device cap interaction in the locked state (top) and in the unlocked state (bottom). In this variant the radially flexible locking arm 11c is part of the device cap 11, and the locking ledges thereof, in the locked state, engage a recess 10c formed in the housing 10.The locking arm may be part of an inner sleeve of the device cap, or may be part of a metallic remover sleeve of the device cap 11, adapted to engage a rigid needle shield 12a sealing the needle of the pre-filled syringe 12.

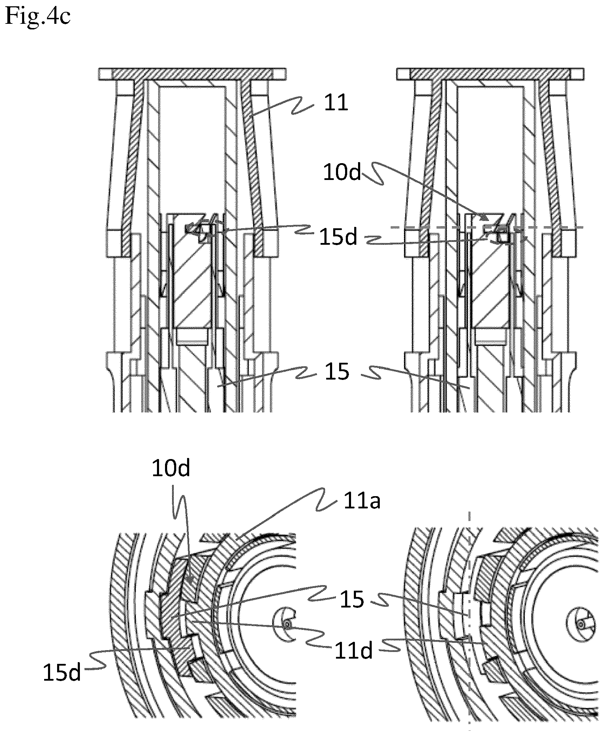

Fig.4c depicts an off-center longitudinal section along a plane tangential to the inner sleeve of the device cap (top) and a cross section perpendicular to the main axis (bottom) of the auto-injector with a third variant of the locking slider - device cap interaction in the locked state (left) and in the unlocked state (right). In this variant, removal of the device cap 11 includes first a rotation by a few degrees as depicted in the bottom drawings, followed by a combined rotational-axial unscrewing movement. The plane of the perpendicular cross section is indicated with a broken line in the top right drawing, and the intersection of the perpendicular and the longitudinal section plane is depicted by a dash-dot line in the bottom right drawing. A rotating cam 11d extending radially outwards from the inner sleeve 11a of the device cap 11 is guided by a cam-path 10d in the housing, wherein a locking cam 15d extending radially inward from the locking slider 15 into the cam path prevents the rotating cam 11d from being rotated in the locked state. In the unlocked state, the locking slider 15 is shifted proximally, and the locking cam 15d is moved out of the blocking engagement with the rotating cam 11d.

Alternative to the shape memory alloy based materials, the actuators may include a magnetic core shifted by the magnetic field of an electromagnet or a solenoid; a DC motor, a stepper motor, or a linear motor, all with or without gearing, Further possibilities include actuators based on a piezo-effect, a bimetal, electroactive polymers, air cushion, or an H2 cell, or a one-time operational melting fuse.

The locking arm or the recess of the previous variants may be part of the cover sleeve instead of the housing. The locking arm may be arranged to flex radially inward upon locking slider retraction and device cap retrieval. The distal head of the locking slider may be positioned, in the locked state, between two off-center flexible locking arms of the device cap engaging two recesses of the housing facing each other. Upon locking slider retraction, these two locking arms may be deflected towards each other in a tangential direction. Alternative variants of the locking slider - device cap interaction include a distally oriented locking arm of the device cap or a distally oriented claw of the remover sleeve abutting a proximally oriented stop surface of the housing, and a locking slider being rotated or shifted distally to urge the locking arm out of engagement with the stop surface. Further concepts involve pivotal levers, or a lock wheel that may be rotated in the unlocked state by less than 180° by the device cap being removed, against a biasing spring.

The electronic unit being integrated in the delivery device may comprise a visual, audible and/or tactile status indicator indicating to a user a status of the system. The status of the system may include a device status of the delivery device or a delivery status of a drug dispensing process. The status indicator may be simple and limited to a few Light Emitting Diodes LEDs in traffic-light colors and/or an audible signal generator for generating language-independent beep sounds or simple melodies. In particular, the status information may include an indication about a lapse of a minimum holding, delay, or dwell time following completion of a substance dispensing activity to inform the user that it is now safe to remove the delivery device. The status indicator may explicitly exclude any advanced human-machine interfacing capability, and be limited to a few, specifically less than ten, messages conveyable to the user. In particular, the delivery device may be devoid of a display, screen, or projector for visually transmitting readable instructions, and likewise exclude an artificial speech assistant for reading out loud the instructions.

While the invention has been described in detail in the drawings and foregoing description, such description is to be considered illustrative or exemplary and not restrictive. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims shall not preclude the existence of further meaningful combinations of these elements or steps.

**LIST OF REFERENCE NUMERALS**

| | | | |
|---|---|---|---|
| 1 | injection device | 12 | prefilled syringe |
| 2 | Gateway | 12a | needle shield |
| 3 | therapy management system | 13 | cover sleeve |
| 4 | Pharmacy | 14 | electronic unit |
| 5 | health care professional | 15 | locking slider |
| 6 | health portal | 15a | base |
| 7 | electronic patient file | 15b | arm |
| 10 | Housing | 15c | tip |
| 10a | locking ledge | 15d | locking cam |
| 10b | locking arm | 16 | actuator bracket |
| 10c | Recess | 16a | actuator arm |
| 10d | cam path | 16b | transverse ledge |
| 11 | device cap | 17a, b | SMA wire |
| 11a | inner sleeve | 18 | anchor |
| 11b | Recess | 18a | blocking surface |
| 11c | locking arm | 18b | pulley |
| 11d | rotating cam | | |

## Claims

1. A delivery device (1) for self-administration of a predefined amount of drug from a reservoir (12) through an outlet, with a device operation lock (10a, 11b; 10c, 11c) preventing a delivery operation of the delivery device as shipped, with a wireless receiver for receiving an unlock command, and with an actuator (16) for unlocking the device operation lock and activated instantaneously upon receipt of the unlock command.

2. The delivery device of claim 1, wherein the delivery device is an auto-injector or a patch-injector.

3. The delivery device of claim 2, wherein the device operation lock includes an outlet cover lock preventing removal of an outlet cover sealing the outlet of the reservoir.

4. The delivery device of claim 3, wherein the outlet cover lock is adapted to be re-locked by the actuator before a delivery operation has occurred.

5. The delivery device of claim 3, wherein the outlet cover lock is a device cap lock including a flexible ledge engaging a counter-ledge and prevented by a locking slider (15) from disengaging.

6. The delivery device of one of claims 1 to 5, comprising a wireless transmitter adapted to send an unlock request message.

7. The delivery device of claim 6, comprising a device-activation sensor to detect a user-initiated preparation of the delivery device.

8. The delivery device of one of claims 1 to 7, comprising drug delivery sensor means to monitor and report a delivery operation of the delivery device.

9. The delivery device of one of claims 1 to 8, comprising an actuator based on a shape-memory-alloy element (17a).

10. A method of unlocking a delivery device with a device operation lock preventing a delivery operation of the delivery device as shipped, for self-administration by a patient, of a predetermined quantity of drug from a reservoir through an outlet of the delivery device, comprising:
- establishing a communication between the patient and a Health Care Professional HCP or a Health Care Expert System HCES, and inquiring about an intended self-administration;
- confirming, by the HCP or HCES, suitability of the intended self-administration by dispatching an unlock command;
- receiving, by a wireless receiver of the delivery device, the unlock command, and
- activating, instantaneously upon receipt of the unlock command, an actuator of the delivery device to unlock the device operation lock.

11. The method of claim 10, comprising
- assigning, at a therapy management system, the delivery device to the patient; and
- dispatching, by the therapy management system, the unlock command following a confirmation by the HCP and based on the assignment.

12. The method of claim 10, comprising
- detecting, by a device-activation sensor of the delivery device, a user-initiated preparation of the delivery device,
- sending, by a wireless transmitter of the delivery device, an unlock request message to the HCP or HCES, and
- contacting, by the HCP or HCES, the patient.

13. The method of claim 10, comprising
- re-locking the device operation lock by the actuator of the delivery device if no delivery operation has occurred within an unlock interval following receipt of the unlocking command.
